# EUROPÄISCHE PATENTSCHRIFT

(11) **EP 2 811 937 B1**
(45) Veröffentlichungstag und Bekanntmachung des Hinweises auf die Patenterteilung: **01.04.2020**
(21) Anmeldenummer: 13703407.0
(22) Anmeldetag: 08.02.2013
(51) Int. Cl.: A61F 2/07, A61F 2/954, A61B 17/068, A61F 2/95

(54) **STENTGRAFT MIT FIXIERELEMENTEN UND EINFÜHRSYSTEM**
STENT GRAFT WITH FIXING ELEMENTS AND INSERTION SYSTEM
GREFFON DE STENT MUNI D'ÉLÉMENTS DE FIXATION ET D'UN SYSTÈME D'INSERTION

(30) Priorität: 10.02.2012 DE 102012101103
(43) Veröffentlichungstag der Anmeldung: 17.12.2014
(73) Patentinhaber: JOTEC GmbH, 72379 Hechingen (DE)
(72) Erfinder: BARTHOLD, Franz-Peter, 72336 Balingen (DE); CENTOLA, Marcos, CEP 15025010 S.J.Do Rio Preto (BR); WOERNE, Christian, 73760 Ostfildern (DE)
(74) Vertreter: Witte, Weller & Partner Patentanwälte mbB
(86) Internationale Anmeldenummer: PCT/EP2013/052584
(87) Internationale Veröffentlichungsnummer: WO 2013/117718

(56) Entgegenhaltungen:
- WO-A2-02/083038
- US-A- 5 873 906
- US-A1- 2006 095 119
- US-A1- 2007 043 425

## Beschreibung

Die vorliegenden Erfindung betrifft einen Stentgraft mit einem hohlzylindrischen Körper mit einer Blutseite und einer Gefäßseite, wobei der Stentgraft einen ersten Stentgraftabschnitt aufweist mit einem selbstexpandierenden Stent aus in seiner Längsrichtung hintereinander angeordneten Ringen aus mäanderförmig umlaufenden Stützen und mit einem an den Ringen befestigten und diese auf der Gefäßseite des hohlzylindrischen Körpers verbindenden ersten Prothesenmaterial.

Derartige Stentgrafts sind im Stand der Technik hinreichend bekannt. Diese auch als endovaskuläre oder endoluminale Stentgrafts oder kurz Stents bezeichnete Gefäßstents werden in Blutgefäße implantiert, welche beispielsweise auf Grund von Krankheiten oder Ähnlichem verletzt, aneurysmatisch aufgeweitet oder in ihrem Lumen verschlossen sind, wodurch die Gefäße in ihrer Funktion stark beeinträchtigt sind oder die Gefahr einer Gefäßruptur besteht. Im Stand der Technik sind verschiedene implantierbare Stentvorrichtungen bekannt, die Blutgefäße, beispielsweise Arterien, nach ihrer Implantation offen halten oder Aufweitungen vom Blutstrom abgrenzen. Solche Stents haben in der Regel einen röhrenförmigen bzw. hohlzylindrischen Körper, der in das Gefäß eingeführt und an der entsprechenden Stelle fixiert wird, um das Lumen des Gefäßes aufrecht zu erhalten.

So sind im Stand der Technik beispielsweise Stentgrafts bekannt, die ein Drahtgerüst aus einem selbstexpandierenden Material, bspw. Nitinol, aufweisen, wobei das Drahtgerüst darüber hinaus mit einem Textil- oder PTFE-Schlauch verbunden sein kann.

Zur Implantation wird der Stent oder Stentgraft radial zusammengedrückt, so dass sich seine Querschnittsfläche deutlich verringert und er einfach in das Gefäß eingeführt werden kann. Auf Grund der Federwirkung des Metallrahmens bzw. der Metallstents expandiert der Stent wieder in seine ursprüngliche Form und spannt dabei seine Mantelfläche auf, die sich innen in dem Blutgefäß verklemmt.

Zur Implantation werden die Stents radial zusammengefaltet und dann mit Hilfe von endoluminal vorgeschobenen Kathetern in das Blutgefäß eingeführt und lagerichtig in dem Gefäß positioniert. Die richtige Lage des Stents kann dabei beispielsweise über Röntgenmarker kontrolliert werden. Damit die Stents während der Positionierung im zusammengefalteten Zustand verbleiben, sind sie üblicherweise in einer Hülse bzw. in einem hülsenartigen Schlauch angeordnet, der den Stent radial nach innen drückt, bzw. komprimiert. Diese sog. Rückzugshülle wird nach dem Positionieren des Stents im Gefäß zurückgezogen, wobei der Stent axial von einem Anschlag-/Schiebeelement gehalten wird, das auch als Pusher bezeichnet wird. Der Pusher liegt dabei in Anlage mit dem Stent und hält diesen in seiner axialen Lage, während die auch den Pusher umgebende Rückzugshülle von dem Stent abgezogen wird, welcher sich dabei expandieren und sich in dem Blutgefäß verklemmen kann.

Bei der Freisetzung eines selbstexpandierenden Stents oder Stentgrafts muss der Arzt dabei oft einen erheblichen Kraftaufwand am Zuggriff des Hüllschlauchs und dem Griff zur Positionierung des Implantats, der mit dem Pusher verbunden ist, aufwenden. Abgesehen von dem Kraftaufwand ist ein weiterer kritischer Punkt oftmals, dass der Stent, sofern er einmal freigesetzt ist, nicht mehr rotiert oder im Gefäß bewegt oder verschoben werden kann, um ihn, falls notwendig korrekt zu platzieren, da ansonsten die Gefahr einer Verletzung des Gefäßes besteht.

Aus der DE 103 35 948 B3 ist ein Stent bekannt, dessen Stützgerüst von einem Faden umschlungen und komprimiert wird. Die Fadenenden werden über eine Umlenkung von außen in das Stützgerüst geführt und dort gekoppelt.

Die WO 2011/063972 A1 offenbart ein Einführsystem zum Einbringen eines medizinischen Implantats in ein Gefäß eines Patienten, wobei mindestens ein Zugfaden eingesetzt wird, mit welchem der Durchmesser eines Implantats geändert werden kann.

Ferner beschreibt die US 2007/0100427 A1 eine Vorrichtung mit einer endoluminalen Prothese, die zwei fadenähnliche Verbindungselemente aufweist, die die Prothese im distalen und proximalen Bereich umfassen, und die durch Verkürzen die Prothese komprimieren können.

Schließlich beschreibt die US 5,776,186 eine Einführvorrichtung für Stentgraft-Systeme, die zwei Nitinol-Drahtschleifen aufweisen, die das Gerüst des Stentgrafts bilden, und über welche nach Zurückziehen des den Stentgraft komprimierenden Einführkatheter der Stentgraft in seine expandierte Form gebracht wird.

Aus der EP 1 964 532 A2 und der EP 1 117 341 B1 sind bspw. ein Einführsystem bekannt, das ein schrittweises Freisetzen eines durch einen Hüllschlauch komprimiert gehaltenen Stents/Stentgrafts ermöglicht. Ferner sind im Stand der Technik sogenannte Pistolengriffe bekannt, die dem Arzt eine kräfteschonende Freisetzung eines ebenfalls durch einen Hüllschlauch komprimiert gehaltenen Stentgrafts ermöglichen sollen.

Die geschilderten Einführsysteme haben jedoch gerade den Nachteil, dass es mit diesen nicht oder kaum möglich ist, den Stent, bevor er endgültig freigesetzt wird, so zu platzieren - d.h. zu rotieren oder zu verschieben -, dass er korrekt zu liegen kommt. Dies ist insbesondere bei Stents/Stentgrafts mit Seitenästen kritisch, da diese sich ja - nach Freisetzung des Stents/Stentgrafts in die abzweigenden Blutgefäße erstrecken sollen. Die US 5873906 zeigt einen Stentgraft, welcher mittels Schlaufen in einem komprimierten Zustand gehalten werden kann.

Vor diesem Hintergrund ist es Aufgabe der vorliegenden Erfindung, einen Stent/Stentgraft sowie ein alternatives Freisetzungssystem bereitzustellen, mit Hilfe dessen der Stent/Stentgraft einfach komprimiert, eingeführt und derart freigesetzt werden kann, dass die Möglichkeit einer korrekten Platzierung des Stents unmittelbar vor der letztendlichen Freisetzung besteht.

Die Erfindung ist durch Ansprüche 1 und 6 definiert. Erfindungsgemäß wird diese Aufgabe gelöst durch eine Weiterbildung des eingangs erwähnten Stentgrafts, wobei dessen erster Stentgraftabschnitt an jedem seiner Ringe jeweils zwei über einen gemeinsamen Fixierbereich angebrachte schlaufenförmige Fixierelemente aufweist, die derart an den Stützen der Ringe angebracht sind, dass durch deren entgegengesetztes Führen um den hohlzylindrischen Körper herum der Stentgraft über seine gesamte Länge komprimierbar ist.

Die Aufgabe wird ferner gelöst durch ein Einführsystem für einen wie oben beschriebenen Stentgraft, der zum Komprimieren und Einführen des Stentgrafts in das Blutgefäß ferner zumindest ein Stiftelement aufweist, das zum auffädelnden Aufnehmen und temporären Fixieren der den Stentgraft komprimierenden schlaufenförmigen Fixierelemente ausgebildet ist.

Die der Erfindung zugrunde liegende Aufgabe wird auf diese Weise vollkommen gelöst. Mit dem erfindungsgemäßen Stentgraft bzw. dem erfindungsgemäßen Einführsystem ist es möglich, den Stentgraft korrekt zu platzieren und ggf. nach dessen Platzierung noch zu rotieren, um eventuell vorgesehene Seitenäste des Stentgrafts in abzweigende Gefäße genau zu platzieren. Auch wird eine schrittweise Freisetzung des Stentgrafts erreicht, dessen einzelne Freisetzungsschritte gezielt kontrolliert werden können.

Mit den schlaufenförmigen Fixierelementen, die an den Stützen der Stentringe angebracht sind, ist es möglich, den Stentgraft zu komprimieren. Die beiden schlaufenartigen Fixierelemente, vorliegend und durchgehend durch die Anmeldung auch synonym mit "Schlaufen" bezeichnet, sind bei dem erfindungsgemäßen Stentgraft an dessen Stentringen bzw. an dessen Stützen angebracht, vorzugsweise angenäht. Die schlaufenförmigen Fixierelemente sind dabei in einem gemeinsamen Fixierbereich an den Stützen angebracht, vorzugsweise angenäht; dieser gemeinsame Fixierbereich kann dabei über einen Abschnitt der Stütze führen oder aber punktförmig sein. Zum Komprimieren des Stentgrafts wird eine Schlaufe der beiden schlaufenförmigen Fixierelemente dabei - bezogen auf den Umfang des Stentgrafts bzw. des hohlzylindrischen Körpers - rechts herumgeführt, und eine Schlaufe links herum; die beiden Schlaufen "treffen" sich dann auf der im Umfang des hohlzylindrischen Körpers dem Fixierpunkt der Fixierelemente gegenüberliegenden Stelle, wo sie übereinandergelegt werden können. Dadurch kann der Stentgraft in dem Bereich der Fixierelemente "zusammengebunden" und dadurch komprimiert werden.

Die schlaufenförmigen Fixierelemente sind dabei vorzugsweise aus einem fadenartigen Material, das üblicherweise in der Medizin eingesetzt wird; bspw. kann das gleiche Material eingesetzt werden, wie es verwendet wird, um die Stentringe an dem Prothesenmaterial zu befestigen; dieses ist vorzugsweise ein Kunststofffaden, vorzugsweise aus Polyester, Polyamid, Polytetraflourethylen oder ultrahochmolekulargewichtiges Polyethylen (UHMPE) oder aus Mischungen davon, bzw. weist die genannten Materialien auf.

Bei dem erfindungsgemäßen Stentgraft ist dabei vorgesehen, dass der Stentgraft an jedem seiner Ringe jeweils zwei über einen gemeinsamen Fixierbereich bzw. -punkt angebrachte schlaufenförmige Fixierelemente aufweist.

Dieses Merkmal hat den Vorteil, dass damit der Stentgraft über seine gesamte Länge komprimiert werden kann.

Die schlaufenförmigen Fixierelemente können dabei im Umfang des hohlzylindrischen Körpers des Stentgrafts jeweils gegeneinander versetzt oder in Längsrichtung benachbart hintereinander an den Stützen angebracht sein.

Gemäß einer weiteren Ausführungsform des erfindungsgemäßen Stentgrafts kann der erste Stentgraftabschnitt ferner zumindest einen, bevorzugt zumindest zwei oder drei, Stentseitenast/Stentseitenäste aufweisen, der zur Einbringung in ein vom Blutgefäß abzweigendes Gefäß ausgebildet ist.

Gemäß einer weiteren Ausführungsform weist der Stentgraft noch einen zweiten Stentgraftabschnitt mit einem schlauchförmigen Körper aus einem zweiten Prothesenmaterial auf; dieses Material kann identisch zu dem ersten Prothesenmaterial sein oder aus einem unterschiedlichem Material bestehen oder dieses aufweisen; dabei weist der zweite Stentgraftabschnitt keine Stentringe auf. Ferner kann gemäß einer weiteren Ausführungsform bevorzugt sein, wenn dieser zweite Stentgraftabschnitt zumindest einen Stentseitenast, noch bevorzugter zwei oder drei Seitenäste, aufweist.

Der zweite Stentgraftabschnitt kann insbesondere für die Rekonstruktion von verletzten oder beschädigten Blutgefäßen vorgesehen sein, und wird in der Regel in solchen Geräten derart angenäht, dass die erkrankten bzw. verletzten oder beschädigten und nicht mehr oder nur eingeschränkt funktionsfähigen Bereiche durch den zweiten Stentgraftabschnitt ersetzt werden. Beispielsweise und insbesondere kann der zweite Stentgraftabschnitt für die Rekonstruktion des Aortenbogens, sowie der Aorta ascendens vorgesehen sein, insbesondere wenn dieser Abschnitt drei Seitenäste aufweist, die jeweils für den Truncus brachiocephalicus, die Arteria Carotis communis und die Arteria subclavia sinistra vorgesehen sind, in welche Gefäße die Seitenäste zur Gewährleistung der Blutversorgung dieser im Aortenbogen angehenden Gefäße eingeführt werden.

Wie weiter oben erwähnt betrifft die vorliegende Erfindung auch ein Einführsystem zum Einführen eines selbstexpandierenden Stentgrafts in ein Blutgefäß eines Patienten, wobei ein Stentgraft wie oben beschrieben eingeführt werden soll, wobei das Einführsystem zum Komprimieren und Einführen des Stentgrafts in das Blutgefäß ferner zumindest ein Stiftelement aufweist, das zum auffädelnden Aufnehmen und temporären Fixieren der den Stentgraft komprimierenden schlaufenförmigen Fixierelemente ausgebildet ist, und das ein proximales und ein distales Ende sowie einen zwischen proximalem und distalem Ende gelegenen mittleren Bereich aufweist.

Das bei dem Einführsystem vorgesehene Stiftelement wird also durch die Schlaufen gefädelt, die jeweils, ausgehend von ihrer Fixierstelle, um den Stentgraft herum geführt wurden und diesen dadurch komprimieren. Durch das Auffädeln der Schlaufen werden diese wiederum fixiert, wodurch der Stentgraft insgesamt komprimiert bleibt. In dieser komprimierten Form kann der Stentgraft in das Blutgefäß eingeführt werden.

Konsequenterweise ist demnach bei der Freisetzung bevorzugt, wenn der komprimierte Stentgraft durch Rückziehen des Stiftelements in proximale Richtung und dadurch Lösen der den Stentgraft komprimiert gehaltenen Schlaufen freisetzbar ist.

Vorliegend wird mit "proximaler Richtung" dabei die Richtung angegeben, die zum Anwender hinführt; ferner wird unter "Stiftelement" jedes längliche, stiftförmige Element verstanden, das lang genug ist und dessen Durchmesser so bemessen ist, um in zumindest einige der an dem Stentgraft vorgesehenen Schlaufen aufgefädelt werden zu können. Folglich weist das Stiftelement ein proximales Ende und ein distales Ende und einen proximalen und distalen Bereich auf, sowie ein zwischen diesen beiden Enden/Bereichen gelegener mittlerer Bereich, wobei das proximale Ende dem Anwender bzw. dem das Einführsystem Handhabenden am nächsten ist. Die Stiftelemente sind dabei vorzugsweise aus Edelstahl, können aber aus jedem anderen beliebigen Material sein, das für die vorliegenden Zwecke geeignet ist und eine gewisse bzw. hinreichende Steifigkeit bietet, um die Schlaufen und dadurch auch den Stentgraft fixiert zu halten.

Beim Zurückziehen des Stiftelements werden also schrittweise die Schlaufen wieder freigegeben, wodurch diese nicht mehr auf der der Fixierstelle entgegengesetzten Stelle im Umfang des Stents zusammengehalten werden, und der Stent dadurch nicht mehr komprimiert und als Konsequenz freigesetzt wird.

Gemäß einer weiteren Ausführungsform des erfindungsgemäßen Einführsystems weist dieses mehrere Stiftelemente unterschiedlicher Länge auf; diese sind derart ausgebildet und durch auffädelndes Aufnehmen der schlaufenförmigen Fixierelemente zum Komprimieren und Freisetzen des Stentgrafts angebracht, dass durch ein Zurückziehen der unterschiedlich langen Stiftelemente in proximaler Richtung unterschiedliche Abschnitte des Stents freisetzbar sind.

Vorzugsweise werden die Stiftelemente dabei mit ihrem proximalen Ende durch ein Mitnahmeelement geführt, durch dessen Bewegen in proximale Richtung die Stiftelemente aufgrund ihrer unterschiedlichen Länge nacheinander in proximale Richtung mitgeführt werden. Dadurch können vorteilhafterweise Stentbereiche, die durch unterschiedlich lange Stiftelemente fixiert/komprimiert werden, zu unterschiedlichen Zeitpunkten und gezielt freigesetzt werden.

Wenn mehrere Stiftelemente unterschiedlicher Länge bei dem erfindungsgemäßen Einführsystem vorgesehen sind, ist bevorzugt, wenn die schlaufenförmigen Fixierelemente an den Ringen des Stents jeweils versetzt gegeneinander angebracht sind. Bei dieser Ausführungsform greift dann jedes Stiftelement in ein schlaufenförmiges Fixierelementenpaar. Sind die schlaufenförmigen Fixierelemente jeweils benachbart in Längsrichtung des Stentgrafts an den Ringen angebracht, so ist bevorzugt, wenn ein Stiftelement eingesetzt wird; in diesem Fall werden alle schlaufenförmigen Fixierelementpaare von dem einen Stiftelement aufgefädelt.

Gemäß einer weiteren Ausführungsform ist bei dem erfindungsgemäßen Einführsystem ferner zusätzlich ein Hüllschlauch vorgesehen ist, der über den den Stent in Zusammenwirkung mit den schlaufenförmigen Fixierelementen komprimierenden Stiftelementen angeordnet ist.

Mit dieser Ausführungsform wird die Möglichkeit geschaffen, sowohl einen Hüllschlauch als auch den Freisetzungsmechanismus aus Stiftelement/schlaufenförmigen Fixierelementen zur Freisetzung des Stentgrafts zu nutzen. Dieser "kombinierte" Freisetzungsmechanismus ist insbesondere bei Stentgrafts von Vorteil, die Seitenäste bzw. vom Hauptkörper des Stentgrafts abgehende Ärmchen besitzen: hier kann der gesamte Stentgraft zunächst über das Rückziehen des Hüllschlauchs generell freigesetzt werden, wobei der Stentgraftbereich mit den Ärmchen noch durch den Mechanismus aus Stiftelement und schlaufenförmigen Fixierelementen komprimiert bleibt. Dadurch können die Seitenäste/abgehenden Ärmchen erst korrekt, d.h. in die abzweigenden Gefäße platziert werden, bevor auch dieser Bereich dann durch Zurückziehen des oder der Stiftelemente freigesetzt wird. In diesem Fall kann das Stiftelement auch, d.h. zusätzlich zur Durchführung durch die Schlaufen, durch das Ärmchen geführt werden.

Bei einer bevorzugten Ausführungsform ist ferner vorgesehen, wenn das Stiftelement mit seinem proximalen Ende um eine in einem Handgriff oder Gehäuse des Einführsystems vorgesehene Rolle geführt wird.

Diese Ausführungsform hat den Vorteil, dass nach Rückziehen des Hüllschlauchs, der vorzugsweise mit dem Handgriff gekoppelt ist und der durch dessen Ziehen in proximale Richtung vom Stentgraft zurückgezogen wird, der Freisetzungsweg für die Stiftelemente immer noch lang genug sein wird, um auch die Stifte aus den Schlaufen zu führen und den Stentgraft vollständig freizusetzen.

Es versteht sich, dass das Einführsystem gemäß der vorliegenden Erfindung darüber hinaus auch jegliche weiteren, für Stent-Einführsysteme übliche Merkmale wie bspw. einen Pusher, Katheterrohr, etc. aufweist; für den Fachmann ist ausgehend von der hierin offenbarten Lehre ohne Weiteres erkennbar, welche Merkmale und Eigenschaften das Einführsystem noch aufweisen kann, um für den jeweiligen Einsatz funktionsfähig zu sein. Beispielhaft wird auf die EP 1 964 532 verwiesen.

Weitere Vorteile ergeben sich aus der nachstehenden Beschreibung vorteilhafter Ausführungsformen und den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den nachstehenden Figuren dargestellt und werden in Bezug auf diese im Folgenden näher beschrieben. Es zeigen:
- Fig. 1:: eine schematische, ausschnittsweise Darstellung eines ersten Ausführungsbeispiels des erfindungsgemäßen Stentgrafts;
- Fig. 2:: eine schematische, ausschnittweise Darstellung eines weiteren Ausführungsbeispiels des erfindungsgemäßen Stentgrafts;
- Fig. 3:: einen vergrößerten Ausschnitt einer weiteren Ausführungsform des erfindungsgemäßen Stentgrafts;
- Fig. 4:: eine Darstellung des in Fig. 1 gezeigten Ausführungsbeispiels des erfindungsgemäßen Stentgrafts in komprimierter Form mit eingefädeltem Stiftelement;
- Fig. 5: einen Ausschnitt einer Ausführungsform eines erfindungsgemäßen Stentgrafts; und
- Fig. 6: eine schematische Darstellung einer Ausführungsform des erfindungsgemäßen Freisetzungssystems des erfindungsgemäßen Stentgrafts.

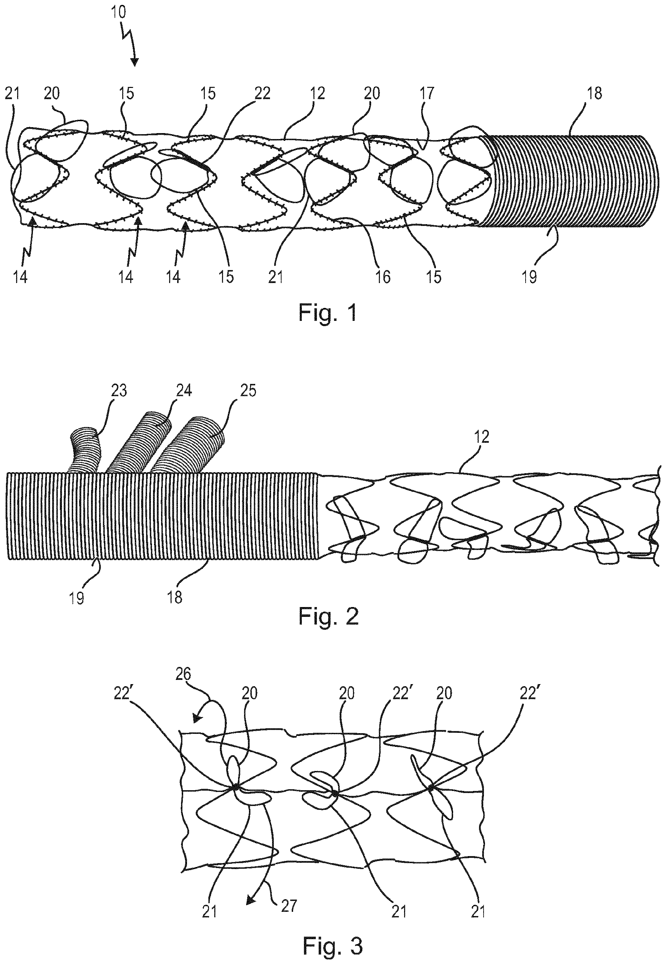

In Fig. 1 ist mit 10 allgemein eine erste Ausführungsform eines erfindungsgemäßen Stentgrafts gezeigt, der einen hohlzylindrischen Körper 11 mit einem ersten Stentgraftabschnitt 12 mit einem selbstexpandierenden Stent aus in seiner Längsrichtung hintereinander angeordneten Ringen 14 aus mäanderförmig umlaufenden Stützen 15 aufweist. Die Stützen 15 sind auf der Außenseite des hohlzylindrischen Körpers 11 durch ein an den Stützen 15 über Nähte 16 befestigtes Prothesenmaterial 17 miteinander verbunden. Der in Fig. 1 gezeigte Stentgraft weist ferner einem zweiten Stentgraftabschnitt 18 auf; dieser zweite Stentgraftabschnitt 18 weist im Gegensatz zum ersten Stentgraftabschnitt 12 keine Stents aus Ringen mit mäanderförmig umlaufenden Stützen auf, sondern umfasst lediglich ein zweites Prothesenmaterial 19.

Ferner sind in Fig. 1 mit 20, 21 schlaufenförmigen Fixierelemente bzw. Schlaufen gezeigt, die sich paarweise an einer Stütze 15 eines Rings 14 befinden und dort an einem gemeinsamen Fixierbereich 22 angebracht sind; in dem in Fig. 1 gezeigten Beispiel sind die schlaufenförmigen Fixierelemente 20, 21 an die Stützen 15 eines Rings 14 angenäht, und zwar über einen gemeinsamen Abschnitt Stütze 15, der den Fixierbereich 22 darstellt. Die Schlaufen 20, 21 sind dabei gleich bzw. in etwas gleich groß.

In den Figuren sind gleiche Merkmale der verschiedenen Ausführungsformen mit gleichen Bezugszeichen versehen. Die Nähte 16, über die das Prothesenmaterial 17 mit den Ringen 14 des Stents verbunden ist, sind in den Figuren 2 bis 5 aus Gründen der Übersichtlichkeit nicht dargestellt.

Alternativ ist bei der in Fig. 3 ausschnittweise gezeigten Ausführungsform der gemeinsame Fixierbereich 22' der schlaufenförmigen Fixierelemente 20, 21 punktförmig auf den Stützen 15 angebracht. In Fig. 3 sind ferner zwei Pfeile 26 und 27 gezeigt, die die Richtung angeben, in welche die beiden schlaufenförmigen Fixierelemente 20, 21 jeweils geführt werden, um den hohlzylindrischen Körper 11 des Stentgrafts 10 "einzubinden" bzw. zu komprimieren.

Fig. 2 zeigt eine weitere Ausführungsform des erfindungsgemäßen Stentgrafts: Hier weist der zweite Stentgraftabschnitt 18 Seitenäste/Ärmchen 23, 24, 25 auf, die zur Einbringung in vom Hauptgefäße abgehende Seitengefäße vorgesehen sind.

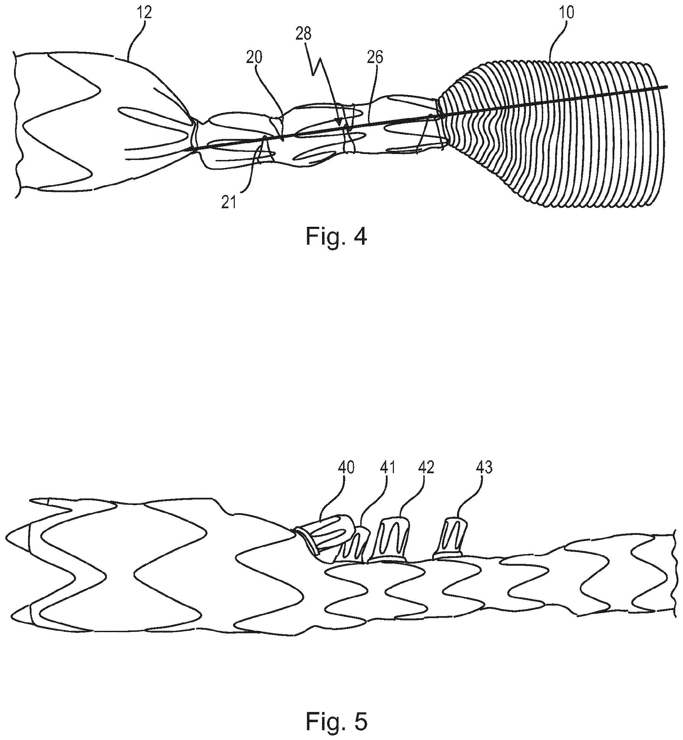

In Fig. 4 ist die in Fig. 1 gezeigte Ausführungsform des erfindungsgemäßen Stentgrafts 10 gezeigt, und zwar in einem teilweise komprimierten Zustand: Hier wurden die schlaufenförmigen Fixierelemente 20, 21, jeweils in verschiedene Umlaufrichtungen um den hohlzylindrischen Körper 11 des Stentgrafts 12 geführt und anschließend ein Stiftelement 26 durch die Schlaufen der schlaufenförmigen Fixierelemente 20, 21 gefädelt. Wie in Fig. 4 zu erkennen ist, treffen sich die Schlaufen, wenn sie jeweils in entgegen gesetzte Richtungen um den Stentgraft geführt werden, auf den den Fixierbereichen 22, 22' im Umfang des Stentgrafts 10 gegenüberliegenden Bereichen 28, wo sie von dem Stiftelement 26 aufgefädelt werden.

Fig. 5 zeigt eine weitere Ausführungsform des erfindungsgemäßen Stentgrafts, wobei hier der ersten Stentgraftabschnitt 12 Ärmchen bzw. Seitenäste 40, 41, 42 und 43 aufweist, die für vom Hauptgefäß abgehende Seitengefäße vorgesehen sind.

Die in Fig. 5 gezeigte Ausführungsform kann bspw. für den Einsatz in der abdominellen Aorta vorgesehen sein, so dass der Stentgraft 10 mit seinem hohlzylindrischen Körper 11 derart in die Aorta abdominalis eingeführt wird, dass die Seitenäste 40, 41, 42, und 43 bspw. in die Abgänge für Truncus coeliacus, Arteria Mesenterica Superior und beide Renalarterien (Arteriae renales) gelegt werden können. Bei dieser Ausführungsform kann das Stiftelement 26 durch eines der Ärmchen 40, 41, 42, 43 und durch die den Stentgraft 10 komprimierenden schlaufenförmigen Fixierelemente 20, 21 geführt werden.

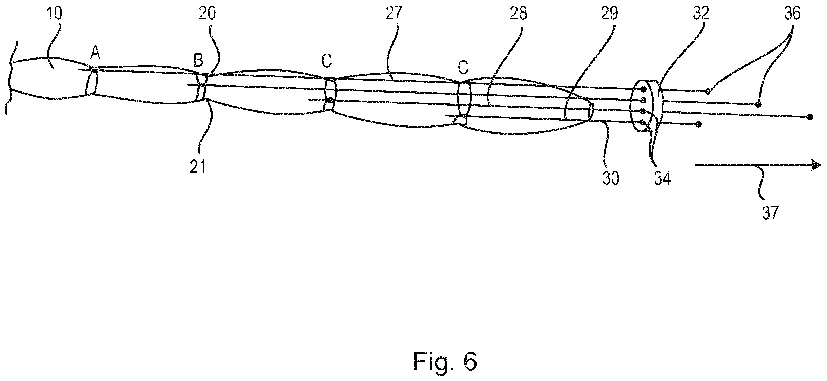

Fig. 6 zeigt schematisch eine weitere Ausführungsform eines erfindungsgemäßen Freisetzungssystems. Auch die in Fig. 6 gezeigte Ausführungsform des erfindungsgemäßen Stentgrafts 10 weist schlaufenförmige Fixierelemente 20, 21 auf, die jeweils in unterschiedlichen Richtungen um den Stentgraft 10 gewickelt wurden. Ferner sind mehrere Stiftelemente 27, 28, 29, 30 vorgesehen, die jeweils in ein Schlaufenpaar 20, 21 aufgefädelt sind: So ist das mit A bezeichnetet Schlaufenpaar 20,21 durch das Stiftelement 27 aufgefädelt, das mit B bezeichnete Schlaufenpaar 20, 21 durch das Stiftelement 28, das mit C bezeichnete Schlaufenpaar durch das Stiftelement 29 und das mit D bezeichnete Schlaufenpaar durch das Stiftelement 30.

Die Stiftelemente 27, 28, 29 und 30 sind über ihr proximales, das heißt dem Anwender näher gelegenes Ende durch ein scheibenförmiges Mitnahmeelement 32 geführt, das vier Bohrungen 34 aufweist, durch welche die Stiftelemente 27, 28, 29 und 30 jeweils geführt sind. Am äußersten Ende des proximalen Bereichs der Stiftelemente 27, 28, 29 und 30 sind jeweils Verdickungen bzw. Stoppelemente 36 vorgesehen, deren Durchmesser größer als der Durchmesser der Bohrungen 34 ist.

In Fig. 6 zeigt Pfeil 37 die Richtung an, in die die Stiftelemente und das Mitnahmeelement 32 geführt werden, um den Stentgraft 10 bzw. einzelne Stentgraftbereiche nacheinander freizusetzen: Wird das Mitnahmeelement 32 in proximale Richtung, d.h. zum Anwender hin, bewegt, so zieht es durch den Anschlag der Verdickung/des Stoppelements 36 die Stiftelemente 27, 28, 29, 30 mit, und zwar in der Reihenfolge, wie die Stoppelemente/Verdickungen 36 vom Mitnahmeelement 32 "aufgenommen" werden: In dem in Fig. 6 gezeigten Beispiel wird also zunächst das Stiftelement 30 mitgenommen und in proximale Richtung gezogen, wodurch in dem in Fig. 6 gezeigten Ausführungsbeispiel zunächst der Bereich D des Stentgrafts 10 freigesetzt und expandiert wird; im weiteren Verlauf der Bewegung in proximale Richtung (siehe Pfeil 37) wird das Stiftelement 27 mitgezogen, so dass sich nach Herausziehen des Stiftelements 27 aus dem Schlaufenpaar 20, 21 der Bereich A des Stentgrafts 10 expandieren kann. Schließlich folgen noch die Bereiche B und C, die durch sequentielles Mitnehmen der Stoppelemente 36 der Stiftelemente 28 und 29 und durch Herausfädeln dieser Stiftelemente 28, 29 aus den Schlaufenpaaren 20, 21 expandieren können.

Auf diese Weise können beliebige Bereiche - je nach Anordnung und Auffädelung der Stiftelemente - zu unterschiedlichen Zeiten gezielt freigesetzt werden.

In einer weiteren, nicht dargestellten Ausführungsform wird das Stiftelement 26 - bzw. die Stiftelemente 27, 28, 29, 30 - über dessen/deren proximales Ende/n auf eine in einem Handgriff oder Gehäuse zur Handhabung des Freisetzungs-/Einführsystems befindliche Rolle geführt und aufgewickelt; diese Ausführungsform ist insbesondere dann von Vorteil, wenn, wie weiter oben beschrieben, neben dem erfindungsgemäßen Freisetzungssystem noch ein Hüllschlauch (in den Figuren nicht gezeigt) vorliegt. Dieser Hüllschlauch ist über dem erfindungsgemäßen Freisetzungssystem aus schlaufenförmigen Fixierelementen 20, 21 und Stiftelementen 26, 27, 28, 29, 30 angeordnet und wird konsequenterweise vor dem erfindungsgemäßen Freisetzungssystem vom Stentgraft 10 zurückgezogen. Da der Hüllschlauch oftmals mit dem Handgriff/Gehäuse gekoppelt ist, und dieser/dieses daher bereits in proximale Richtung geführt ist, bliebe regelmäßig nur ein kurzer Freisetzungsweg für die Stiftelemente 26, 27, 28, 29, 30, die ja dann ebenfalls in die proximale Richtung gezogen werden müssten. Durch das Aufrollen bzw. Aufwickeln der Stiftelemente 26, 27, 28, 29, 30 im Handgriff ist gewährleistet, dass der Freisetzungsweg hinreichend lang genug ist, um auch die Stiftelemente 26 bzw. 27, 28, 29, 30 aus den schlaufenförmigen Fixierelementen 20, 21, herauszuziehen und den Stentgraft 10 dadurch insgesamt freizusetzen.

## Patentansprüche

1. Stentgraft (10) zum Einführen in ein Blutgefäß eines Patienten, mit einem hohlzylindrischer Körper (11) mit einer Blutseite und einer Gefäßseite, wobei der Stentgraft (10) Folgendes aufweist:
einen ersten Stentgraftabschnitt (12) mit einem selbstexpandierenden Stent aus in seiner Längsrichtung hintereinander angeordneten Ringen (14) aus mäanderförmig umlaufenden Stützen (15) und mit einem an den Ringen (14) befestigten und diese auf der Gefäßseite des hohlzylindrischen Körpers (11) verbindenden ersten Prothesenmaterial (17),
**dadurch gekennzeichnet, dass** der erste Stentgraftabschnitt (12) an jedem seiner Ringe (14) jeweils zwei über einen gemeinsamen Fixierbereich (22) angebrachte schlaufenförmige Fixierelemente (20, 21) aufweist, die derart an den Stützen (15) der Ringe (14) angebracht sind, dass durch deren entgegen gesetztes Führen um den hohlzylindrischen Körper (11) herum dieser über seine gesamte Länge komprimierbar ist.

2. Stentgraft (10) nach Anspruch 1, **dadurch gekennzeichnet, dass** die Fixierelemente (20, 21) im Umfang des hohlzylindrischen Körpers (11) des Stentgrafts (10) versetzt oder benachbart hintereinander an den Stützen (15) angebracht sind.

3. Stentgraft (10) nach einem der vorstehenden Ansprüche, **dadurch gekennzeichnet, dass** der erste Stentgraftabschnitt (12) ferner zumindest einen Stentseitenast (40, 41, 42, 43) aufweist, der zur Einbringung in ein vom Blutgefäß abzweigendes Gefäß ausgebildet ist.

4. Stentgraft (10) nach einem der vorstehenden Ansprüche, **dadurch gekennzeichnet, dass** er einen zweiten Stentgraftabschnitt (18) aufweist, mit einem schlauchförmigen Körper aus einem zweiten Prothesenmaterial (19), wobei der zweite Stentgraftabschnitt (18) keine Stentringe aufweist.

5. Stentgraft (10) nach Anspruch 4, **dadurch gekennzeichnet, dass** der zweite Stentgraftabschnitt (18) zumindest einen Stentseitenast (23, 24, 25) aufweist.

6. Einführsystem zum Einführen eines selbstexpandierenden Stentgrafts (10) in ein Blutgefäß eines Patienten, **dadurch gekennzeichnet, dass** das Einführsystem einen Stentgraft (10) nach einem der Ansprüche 1 bis 5 aufweist, sowie zum Komprimieren und Einführen des Stentgrafts (10) in das Blutgefäß ferner zumindest ein Stiftelement (26; 27; 28; 29; 30), das zum auffädelnden Aufnehmen und temporären Fixieren der den Stentgraft (10) komprimierenden schlaufenförmigen Fixierelemente (20, 21) ausgebildet ist, wobei das Stiftelement (26; 27; 28; 29; 30) ein proximales und ein distales Ende, sowie einen dazwischen liegenden mittleren Abschnitt aufweist.

7. Einführsystem nach Anspruch 6, **dadurch gekennzeichnet, dass** der komprimierte Stentgraft (10) durch Rückziehen des Stiftelements (26; 27; 28; 29; 30) in proximale Richtung und dadurch Lösen der den Stentgraft (10) komprimiert gehaltenen schlaufenförmigen Fixierelemente (20, 21) freisetzbar ist.

8. Einführsystem nach Anspruch 6 oder 7, **dadurch gekennzeichnet, dass** mehrere Stiftelemente (26; 27; 28; 29; 30) unterschiedlicher Länge zum Freisetzen unterschiedlicher Stentgraftabschnitte vorgesehen sind.

9. Einführsystem nach einem der Ansprüche 6 bis 8, **dadurch gekennzeichnet, dass** ferner zusätzlich ein Hüllschlauch vorgesehen ist, der über den den Stentgraft (10) in Zusammenwirkung mit den schlaufenförmigen Fixierelementen (20, 21) komprimierenden Stiftelementen (26; 27; 28; 29; 30) angeordnet ist.

10. Einführsystem nach einem der Ansprüche 6 bis 9, **dadurch gekennzeichnet, dass** es ferner einen Handgriff aufweist, sowie im Handgriff eine Rolle, über welchen das Stiftelement (26; 27; 28; 29; 30) mit seinem proximalen Ende führbar ist.

## Claims

1. A stent graft (10) for inserting into a blood vessel of a patient, having a hollow cylindrical body (11) with a blood side and a vessel side, wherein the stent graft (10) has the following:
a first stent graft portion (12) with a self-expanding stent composed of successive rings (14) of meandering circumferential supports (15) in its longitudinal direction, and with a first prosthesis material (17) secured on the rings (14) and connecting these on the vessel side of the hollow cylindrical body (11),
**characterized in that**, on each of its rings (14), the first stent graft portion (12) has two loop-shaped fixing elements (20, 21) which are attached via a common fixing area (22) and which are attached to the supports (15) of the rings (14) in such a way that, due to the fact that they are guided in opposite directions around the hollow cylindrical body (11), the latter is compressible.

2. The stent graft (10) as claimed in claim 1, **characterized in that** the fixing elements (20, 21) in the circumference of the hollow cylindrical body (11) of the stent graft (10) are attached to the supports (15) in a manner offset or adjacent to and behind one another.

3. The stent graft (10) as claimed in one of the preceding claims, **characterized in that** the first stent graft portion (12) moreover has at least one stent side branch (40, 41, 42, 43) designed to be introduced into a vessel branching off from the blood vessel.

4. The stent graft (10) as claimed in one of the preceding claims, **characterized in that** it has a second stent graft portion (18) with a tubular body composed of a second prosthesis material (19), wherein the second stent graft portion (18) has no stent rings.

5. The stent graft (10) as claimed in claim 4, **characterized in that** the second stent graft portion (18) has at least one stent side branch (23, 24, 25).

6. An insertion system for inserting a self-expanding stent graft (10) into a blood vessel of a patient, **characterized in that** the insertion system has a stent graft (10) as claimed in one of claims 1 through 5, and, in order to compress and insert the stent graft (10) into the blood vessel, moreover has at least one pin element (26; 27; 28; 29; 30) designed to be threaded through and temporarily fix the loop-shaped fixing elements (20, 21) compressing the stent graft (10), wherein the pin element (26; 27; 28; 29; 30) has a proximal end and a distal end, and a middle portion lying between these ends.

7. The insertion system as claimed in claim 6, **characterized in that** the compressed stent graft (10) can be released by pulling the pin element (26; 27; 28; 29; 30) back in the proximal direction and thereby releasing the loop-shaped fixing elements (20, 21) that have kept the stent graft (10) compressed.

8. The insertion system as claimed in claim 6 or 7, **characterized in that** several pin elements (26; 27; 28; 29; 30) of different length are provided for releasing different stent graft portions.

9. The insertion system as claimed in one of claims 6 to 8, **characterized in that** a sleeve tube is additionally provided which is arranged over the pin elements (26; 27; 28; 29; 30) compressing the stent graft (10) in cooperation with the loop-shaped fixing elements (20, 21).

10. The insertion system as claimed in one of claims 6 to 9, **characterized in that** it moreover has a handle and, within the handle, a roller via which the pin element (26; 27; 28; 29; 30) can be guided with its proximal end.

## Revendications

1. Greffon d'endoprothèse (10) pour l'insertion dans un vaisseau sanguin d'un patient, comprenant un corps cylindrique creux (11) avec un côté sang et un côté vaisseau, le greffon d'endoprothèse (10) présentant :
une première portion de greffon d'endoprothèse (12) avec une endoprothèse auto-expansible constituée d'anneaux (14) disposés les uns derrière les autres dans sa direction longitudinale, constitués de supports périphériques en forme de méandres (15), et avec un premier matériau de prothèse (17) fixé aux anneaux (14) et reliant ceux-ci sur le côté vaisseau du corps cylindrique creux (11),
**caractérisé en ce que** la première portion de greffon d'endoprothèse (12) présente, au niveau de chacun de ses anneaux (14), à chaque fois deux éléments de fixation en forme de boucle (20, 21) montés par le biais d'une région de fixation commune (22), qui sont montés sur les supports (15) des anneaux (14) de telle sorte que leur guidage dans un sens opposé autour du corps cylindrique creux (11) permette de comprimer celui-ci sur toute sa longueur.

2. Greffon d'endoprothèse (10) selon la revendication 1, **caractérisé en ce que** les éléments de fixation (20, 21) sont montés dans la périphérie du corps cylindrique creux (11) du greffon d'endoprothèse (10) de manière décalée ou juxtaposée les uns derrière les autres au niveau des supports (15).

3. Greffon d'endoprothèse (10) selon l'une quelconque des revendications précédentes, **caractérisé en ce que** la première portion de greffon d'endoprothèse (12) présente en outre au moins une branche latérale d'endoprothèse (40, 41, 42, 43) qui est réalisée de manière à être introduite dans un vaisseau partant du vaisseau sanguin.

4. Greffon d'endoprothèse (10) selon l'une quelconque des revendications précédentes, **caractérisé en ce qu'**il présente une deuxième portion de greffon d'endoprothèse (18) avec un corps en forme de tuyau constitué d'un deuxième matériau de prothèse (19), la deuxième portion de greffon d'endoprothèse (18) ne présentant pas d'anneaux d'endoprothèse.

5. Greffon d'endoprothèse (10) selon la revendication 4, **caractérisé en ce que** la deuxième portion de greffon d'endoprothèse (18) présente au moins une branche latérale d'endoprothèse (23, 24, 25).

6. Système d'insertion pour l'insertion d'un greffon d'endoprothèse auto-expansible (10) dans un vaisseau sanguin d'un patient, **caractérisé en ce que** le système d'insertion présente un greffon d'endoprothèse (10) selon l'une quelconque des revendications 1 à 5, et, pour comprimer et insérer le greffon d'endoprothèse (10) dans le vaisseau sanguin, en outre au moins un élément de tige (26 ; 27 ; 28 ; 29 ; 30) qui est réalisé pour recevoir, en les enfilant et en les fixant temporairement, les éléments de fixation en forme de tuyau (20, 21) comprimant le greffon d'endoprothèse (10), l'élément de tige (26 ; 27 ; 28 ; 29 ; 30) présentant une extrémité proximale et une extrémité distale ainsi qu'une portion centrale située entre elles.

7. Système d'insertion selon la revendication 6, **caractérisé en ce que** le greffon d'endoprothèse (10) comprimé peut être libéré en retirant l'élément de tige (26 ; 27 ; 28 ; 29 ; 30) dans la direction proximale et en desserrant ainsi les éléments de fixation (20, 21) en forme de tuyau maintenant le greffon d'endoprothèse (10) à l'état comprimé.

8. Système d'insertion selon la revendication 6 ou 7, **caractérisé en ce que** plusieurs éléments de tige (26 ; 27 ; 28 ; 29 ; 30) de longueurs différentes sont prévus pour libérer différentes portions du greffon d'endoprothèse.

9. Système d'insertion selon l'une quelconque des revendications 6 à 8, **caractérisé en ce qu'**il est en outre prévu un tuyau d'enveloppement qui est disposé pardessus les éléments de tige (26 ; 27 ; 28 ; 29 ; 30) comprimant le greffon d'endoprothèse (10) par coopération avec les éléments de fixation en forme de tuyau (20, 21).

10. Système d'insertion selon l'une quelconque des revendications 6 à 9, **caractérisé en ce qu'**il présente en outre une poignée et, dans la poignée, un rouleau, par le biais duquel l'élément de tige (26 ; 27 ; 28 ; 29 ; 30) peut être guidé par son extrémité proximale.
